Europäisches Patentamt

European Patent Office

Office européen des brevets

(19)

(11) EP 0 966 933 A1

(12) **EUROPEAN PATENT APPLICATION**

(43) Date of publication:
29.12.1999 Bulletin 1999/52

(51) Int. Cl.[6]: **A61F 5/44**

(21) Application number: **98124492.4**

(22) Date of filing: **28.12.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **26.06.1998 WO PCT/US98/13289**
**26.06.1998 WO PCT/US98/13288**

(71) Applicant:
**THE PROCTER & GAMBLE COMPANY**
**Cincinnati, Ohio 45202 (US)**

(72) Inventors:
 • **D'Acchioli, Vincenzo**
  **65779 Kelkheim am Taunus (DE)**
 • **Palumbo, Gianfranco**
  **61348 Bad Homburg (DE)**

(74) Representative:
**Kohol, Sonia et al**
**Procter & Gamble**
**European Service GmbH**
**Sulzbacher Strasse 40-50**
**65823 Schwalbach am Taunus (DE)**

(54) **Breathable urine collector**

(57)     The present invention relates to a urine management device (10) with an increased level of comfort. The urine management device (10) comprises a bag (11) having an aperture (13), and an anatomically-shaped flange (12), which surrounds the aperture (13). The flange (12) provides for adhesive attachment to the uro-genital area of the wearer. In particular, the bag (11) comprises a wall material which is breathable i.e. moisture vapour permeable. In another aspect of the present invention, the urine management device is used in combination with a disposable diaper.

Fig. 1

EP 0 966 933 A1

## Description

### Field of the Invention

The present invention relates to a urine management device providing improved skin compatibility by the utilisation of a breathable bag material.

### Background of the Invention

Urine management devices are known articles of manufacture that are designed to be worn principally by incontinence sufferers and infants. Such faecal management devices are attached to the uro-genital region of the wearer and are intended to entrap and immediately contain urine and other bodily discharges. As a consequence, these devices are functionally effective in eliminating the problem of smearing on the skin of the wearer; in lessening epidermal irritation; in preventing contamination of articles such as clothing and bedding; and even in preventing the soiling of the carers themselves. Nevertheless, a problem often encountered during the use of such urine management devices is that the constituent material of the outer surface of the devices tends to cause discomfort and in some cases, extreme discomfort to, for example, the bedridden wearer or to the infant.

Typically, the urine management devices are made from a plastic material. For instance, GB 1,092,274 discloses a pediatric urine collector for female use comprising a collector bag of plastic material opening. The base of the opening is provided with a wedge like projection adapted to engage the lower perineal area of the infant. The collector is secured to the body of the wear by adhesive material. GB 2,268,882 discloses a urostomy pouch/bag of plastic material provided with a stomal orifice which is surrounded by a first coupling member, by which the pouch can be releasably affixed to a counterpart coupling member. The pouch may also be provided for use as a kit further comprising an applicator of super absorbent material which can be injected into the pouch by the use of a plunger. EP 140 478 discloses a disposable diaper having a water proof barrier preferably polypropylene or polyethylene formed as a flattened bag having a single opening. The bag is provided with 2 layers filler materials, the upper layer being a wicking material and the lower being a super absorbent material. WO 85/03428 discloses a urine retaining device for male patients comprising a protecting bag having an opening, parallelogram-walls of a water impermeable laminate of polyethylene film and nonwoven provided with an insert of water absorbing material.

Plastic material, while functionally acceptable, is endowed with certain characteristics that are unsatisfactory and disagreeable to the wearer. The feel or texture of plastic material is particularly disturbing from the point of view of skin healthiness. Typically, the skin of incontinence sufferers and infants is especially sensitive and needs to be treated gently and with care. Whilst such devices are only releasably attached to the skin at the uro-genital region of the wearer, during wear the bag portion of the device will come into contact with the skin of the wearer. It has been recognised that the rubbing of plastic material of the bag of urine management devices against the body of a wearer during wear can lead to reddening, skin rashes and perhaps, even lead to a more severe skin irritation. Moreover another problem related to the use of such plastic bag materials is that skin will be occluded causing hot and clammy conditions and skin stickiness in use. Such problems which are even further exacerbated when the device is used in combination with a diaper, may cause such discomfort that the device is ultimately discarded. Therefore, a real consumer need can be identified for a urine management device with skin compatibility benefits and preferably providing superior cushioning qualities and a softer tactile feel.

The present invention addresses this need by providing the wall material of the bag with a breathable material i.e. a material which is moisture vapour permeable, allowing the skin to function normally even when in contact with the bag material, whilst still effectively serving its function to contain excreted matter. Furthermore, the urine management device is aesthetically more pleasing, produces less crinkle during use and results in a high level of wearer and carer satisfaction in relation to skin healthiness.

In another aspect of the present invention, the urine management device with its breathable bag material can be advantageously used with a disposable diaper. In this manner, the constituent material of the outer surface of the urine management device can be capitalised upon to reduce not only the problem of occlusion and epidermal irritations, but also contribute greatly to improved skin healthiness and lead to very satisfied wearers.

### Summary of the Invention

A urine management device (10) constructed in accordance with the present invention comprises a bag (11) having an aperture (21) and a flange (12) surrounding the aperture (21) for adhesive attachment to the skin of the wearer. The flange (12) is attached to the bag. The bag (11) comprises a wall material that is selected such that it is breathable i.e. permeable to moisture vapour, and has a moisture vapour transport rate of at least 250 $g/(m^2.24hrs.)$. The bag material is however more preferably permeable to both air and moisture vapour.

According to another aspect of the present the bag material may further comprise additional components such as odour control active agents. It has been found that this combination of a breathable wall material with an odour control system results in an unexpected increase in the effectiveness of the odour control system.

In another aspect of the present invention, the urine management device is used in combination with a disposable diaper wherein the benefits of a breathable wall material for the bag and a breathable wall material comprising an odour control system are particularly advantageous.

Brief description of the drawings

It is believed that the invention will be better understood from the foregoing description in conjunction with the accompanying drawings in which:

Figure 1 is a plan view of a disposable urine management device of the present invention.

Figure 2 is a side view of the disposable urine management device of Figure 1.

Figure 3 is a cross-sectional view taken along line 3-3 of Figure 1.

Figure 4 is a plan view of another embodiment of a disposable urine management device of the present invention.

Figure 5 is a cross-sectional view taken along line 5-5 of Figure 4.

Figure 6 is a cross-sectional view of another embodiment of a disposable urine management device of the present invention.

Detailed description of the invention

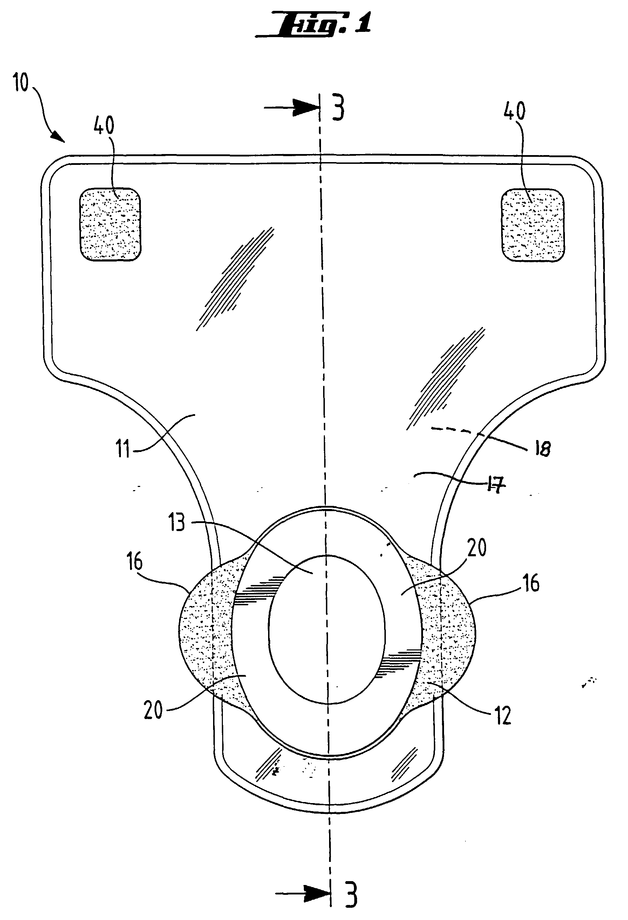

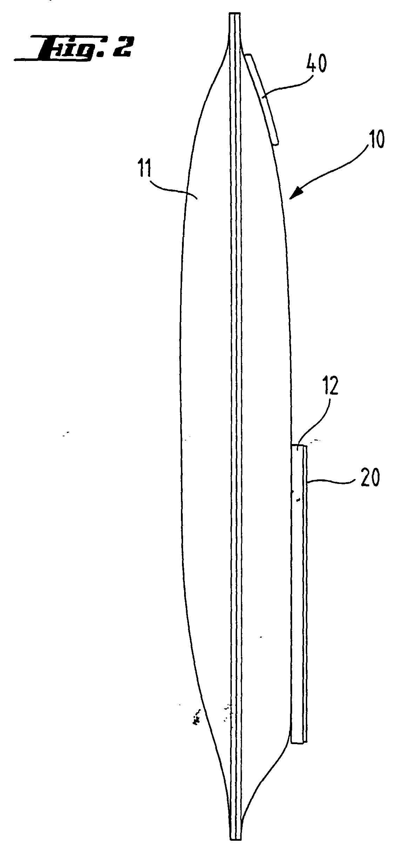

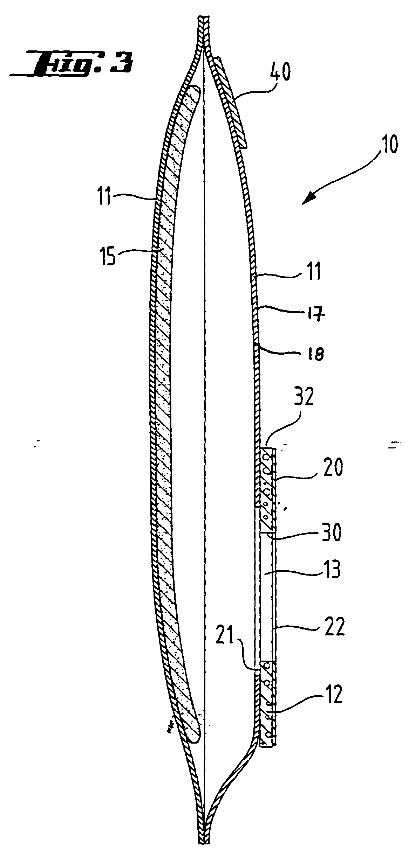

Referring now to figures 1 - 3, there is shown a urine management device (10). Typically urine management devices comprise a bag (11) having an aperture (13) and a flange (12) surrounding the aperture (13) for preferably adhesive attachment to the uro-genital area of a wearer. Any urine management device (10) known in the art can be provided according to the present invention.

According to the present invention, the urine management devices (10) comprise as an essential component a breathable bag wall material. The primary role of the wall material is to prevent the extrudes absorbed and contained in the device from wetting clothing or the diaper that contacts the device. In addition however, the breathable wall material permits the transfer of water vapour and preferably both water vapour and air through it and thus allows the circulation of air into and out of the wall material and the device itself.

In particular, it has been found that in order to avoid the problem of entrapment and condensation of moisture vapour given oft by the body of the wearer and thus, the hot, clammy and uncomfortable conditions that typically occur after a short period of use of such devices; the bag material of the devices of the present invention are breathable.

According to the present invention the wall material of the bag (11) hence has a vapour permeability of greater than 250 g/(m$^2$.24hrs.), preferably greater than 300g/m$^2$/24hrs., more preferably greater than 500 g/m$^2$/24hrs., most preferably greater than 600g/m$^2$/24hrs. In a preferred embodiment of the present invention the wall of the bag also has an air permeability of greater than 50 l/m$^2$/s, more preferably greater than 60l/m$^2$/s, most preferably greater than 70l/m$^2$/s.

According to the present invention any known breathable material or multiple layer breathable material composite may be used in the device provided that the wall material preferably meets the requirement of the liquid permeability test as defined herein. The breathable wall materials of the present invention should hence have a liquid permeability at a 10 ml. load of less than 0.16 g, preferably of less than 0.10 g, more preferably 0 g. The liquid impervious requirement of the wall material to liquids is of more importance for faecal management devices which are designed to worn alone without any additional absorbent article such as a diaper or incontinence device. However for applications where the device is worn in combination with a diaper, the liquid impervious ability of the wall material is not of such critical importance and hence the liquid imperviousness of the wall material can be selected according to the particular needs of the wearer group.

Suitable breathable wall material for use herein include all breathable wall materials known in the art. In principle there are two types of breathable wall materials, single layer breathable wall materials which are breathable and impervious to liquids and wall materials having at least two layers, which in combination provide both breathability and liquid imperviousness.

According to the present invention the wall material of the bag hence comprises at least 1 layer, preferably at least 2 layers and most preferably at least 3 layers. For embodiments wherein the bag material comprises a plurality of layers these layers are typically bonded to one another over substantially the entire surface contact area. The wall material of the bag is selected such that it is breathable. Accordingly the breathability of the bag material may be provided by a single layer or by a combination of layers which together provide the required properties of liquid impermeability and moisture vapour transport.

According to the present invention any known breathable material layer or multiple layer composite may be used as the bag material. Suitable bag materials for use herein comprise at least one gas permeable layer. Suitable gas permeable layers include 2 dimensional, planar micro and macro-porous films, macroscopically expanded films, and monolithic films.

Suitable single layer breathable wall materials for use herein include those described for example in GB A 2184 389, GB A 2184 390, GB A 2184 391, US 4 591 523, US 3 989 867 US 3 156 242 and European Patent Application number 95120653.1.

Suitable dual or multi layer breathable wall materials for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

According to the present invention the breathable wall materials comprises at least one, preferably at least two water vapour permeable layers. Suitable water vapour permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the wall materials comprises a macroscopically expanded film or an apertured formed film, the wall materials will typically have an open area of more than 5 %, preferably from 10 % to 35% of the total wall material surface area.

Suitable 2 dimensional planar layers of the wall materials may be made of any material known in the art, but are preferably manufactured from commonly available polymeric materials. Suitable materials are for example GORE-TEX (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5 mm, wherein the apertures have an average uniform diameter along their length and which do not protrude out of the plane of the layer. The apertured materials for use as a wall materials in the present invention may be produced using any of the methods known in the art such as described in EPO 293 482 and the references therein. In addition, the dimensions of the apertures produced by this method may be increased by applying a force across the plane of the wall materials (i.e. stretching the layer).

Suitable apertured formed films include films which have discrete apertures which extend beyond the horizontal plane of the outer surface (30) of the wall materials towards the interior of the bag thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the interior of the bag. Suitable macroscopically expanded films for use herein include films as described in for example in US 637 819 and US 4 591 523.

Suitable macroscopically expanded films for use herein include films as described in for example US 4 637 819 and US 4 591 523.

Suitable monolithic films include Hytrel$^{TM}$, available from DuPont Corporation, USA, and other such materials as described in Index 93 Congress, Session 7A "Adding value to Nonwovens", J-C. Cardinal and Y. Trouilhet, DuPont de Nemours International S.A, Switzerland.

According to the present invention the wall material may comprise in addition to said water vapour permeable layer, additional layers. Said additional layers may be located on either side of said water vapour permeable layer of the breathable layer. The additional layers may be of any material, such as fibrous layers such as wovens, nonwovens or additional water vapour permeable films.

In addition a further advantage of the breathable bag material is that the generation of malodours associated with occlusive environments are considerably reduced. Moreover the effectiveness of an odour control agents within the device is further improved.

The bag (11) as used herein is a flexible receptacle for the containment of excreted matter. The bag (11) can be provided in any shape or size depending on the intended use thereof, i.e. whether the device is intended for bedridden patients or active patients suffering from incontinence or for infants. For example elongated bags which are principally tubular or rectangular are typically utilised by bedridden patients and elderly incontinence sufferers. For more active wearers whether infants or adults, the faecal management device should preferably be anatomically shaped such that the device follows the contours of the body and can be worn inconspicuously by the wearer under normal garments.

Particularly, preferred shapes are flat circular type bags, flat T shaped bags, cone shaped bags, truncated shaped bags and pyramidal or truncated pyramidal shaped bags. In a most preferred embodiment of the present invention, the bag (11) has a substantially flat T shape.

In addition, the bag (11) is preferably shaped to fit the uro-genital area of the wearer and thereby ensure good contact between the flange and the skin of the wearer. For example the bag (11) may be provided with a neck portion or conduit.

The bag (11) is preferably designed to provide sufficient volume for urine under a variety of wearing conditions, also when worn by a freely moving, i.e. not bedridden wearer.

The bag (11) is designed to safely contain any entrapped material, and is designed of sufficient strength to withstand rupture in use, also when pressure on the bag is exerted in typical wearing conditions, such as sitting.

According to the present invention, depending on the shape of the bag (11) required, the bag (11) may be provided from a unitary piece of material or from a number of separate pieces of material, which may be identical or different and which are sealed at their respective peripheries.

According to the present invention the bag (11) can comprise one or multiple layers, preferably two or three layers. The layer on the inside of the bag, which will typically at least partially come in contact with urine or other bodily excretions is called the inner layer (18). The outermost layer of the bag, which will typically at least partially come in contact with the skin to the wearer and the garments of the wearer, is called the outer layer (17).

The outer layer (17) of the bag is preferably provided with a non-woven layer. Such material layers present an uneven surface to the skin of the wearer and thus reduce significantly the problem of occlusion and greatly improve skin healthiness.

In one preferred embodiment of the present invention the bag comprises two layers. Preferably the outer layer (17) comprises a non-woven layer and the inner layer (18) comprises a monolithic film.

In yet another preferred embodiment of the present invention, the bag (11) comprises three layers, preferably one monolithic film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a monolithic film and the other two layers comprise non-wovens.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the bag. The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-anionic, cationic and non-cationic surfactants, may be added to further enhance softness and surface smoothness.

Furthermore, the non-woven layer may be impregnated with a lotion to provide desirable therapeutic or protective coating lotion benefits. The lotion coating is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

According to a particularly preferred embodiment of the present invention the bag material may further comprise additional components such as odour control agents. It has been found that this combination of a breathable wall material with an odour control system results in an unexpected increase in the effectiveness of the odour control system. It is believed that the synergic odour control performance benefit of a breathable bag wall material in combination with the presence of an odour control agent is due to a number of factors.

Firstly, the breathability of the device results in increased movement of the volatile malodourous compounds. Hence, the amount of actual physical contact between these compounds and the odour control agents increases. Contact between the odour control agents and the malodourous compounds is usually required in order to effectively combat the odourous compound. Frequently, large quantities of the odour control system is required within the urine management devices in order to ensure its effectiveness. This is because the odour control agents do not necessarily contact all the malodourous compounds present. Hence the cost of these products increases, so it is desirable to avoid the necessity of large quantities of the odour control system. In the present invention, the effectiveness of the odour control agent is significantly increased and thus the full capacity of the odour control agent can be utilised and hence less may be required.

Secondly, the breathability of the urine management device wall material reduces the hot humid and anaerobic environment between the skin of the wearer and the surface of the device. This hinders the growth of microorganisms, which are also known to be responsible for the generation of odourous compounds. Thus, the amount of odours associated with the presence of microorganisms is reduced by the urine management devices of the present invention.

Thirdly, the reduction in the hot, humid and occlusive environment between the vicinity of the skin of the wearer and the wearer facing surface of the urine management devices itself also reduces the tendency of the wearer of the product to perspire. Consequently, the amount of associated perspiration related odour will be reduced. Thus, the breathability of the device actually reduces the amount of odour generated within the device. As a result the odour control system works more effectively on the remaining odourous compounds present in the device.

In addition, due to the breathable nature of the device, the malodourous compounds contained therein may, similar to water vapour and air, be more readily exchanged with the environment. Hence, malodourous compounds are able to escape from the device and are dissipated into the surroundings. More importantly, the breathability of the bag wall material also allows the precursors compounds of malodourous compounds present in the device to escape from the device before degradation commences and hence before malodour formation takes place.

Any odour control agent or combinations thereof, known in the art for this purpose may be used herein as an odour control system. The art is replete with descriptions of various odour controlling agents for use in absorbent article in order to address the problem of malodour formation which may all be usefully employed in the present invention. These agents can typically be classified according to the type of odour the agent is intended to combat. Odours may be chemically classified as being acidic, basic or neutral. Acidic odour controlling agents have a pH greater than 7 and typically include sodium carbonates, sodium bicarbonates, sodium phosphates, particularly zinc and copper sulphates. Basic odour controlling agents have a pH of less than 7 and include compounds such as carboxylic acids such as citric acid, laric acid, boric acid, adipic acid and maleic acid.

Neutral odour controlling agents have a pH of approximately 7. Examples of these types of compounds include activated carbons, clays, zeolites, silicas, absorbent gelling materials, (AGM) and starches. Neutral odour control agents and systems are disclosed for example in EPO 348 978, EPO 510 619, WO 91/12029, WO 91/11977, WO 91/12030, WO 81/01643 and WO96/06589. Also cyclodextrin and derivatives thereof may be used as described in US 5429628.

Alternatively, the odour control systems may be categorised with respect to the mechanism by which the malodor detection is reduced or prevented. The above odour control agents typically control odour detection by an absorptive mechanism.

Hence, odour control systems which chemically react with malodourous compounds or with compounds which produce malodourous degradation products thereby generating compounds lacking odour or having an odour acceptable to consumers may also be utilised herein. Suitable agents include chelating agents and may be selected from amino carboxylates such as for example ethylenediamine- tetracetate, as described for example in US 4356190, amino phosphonates such as ethylenediaminetetrakis (methylene- phosphonates), polyfunctionally-substituted aromatic chelating agents as described in US 3 812 044 and mixtures thereof. Without intending to be bound by theory it is believed that the benefit of these compounds is in part due to their exceptional ability to remove iron, copper, calcium, magnesium and manganese ions present in the absorbed fluids and their degradation products by the formation of chelates.

Another suitable odour control system for use herein comprises a buffer system, such as citric acid and sodium bicarbonate, sodium phosphate and sorbic acid buffer systems. Also, buffer systems having a pH of from 7 to 10 as described for example in WO 94/25077 may be useful herein.

An alternative odour control system utilises ion exchange resins such as those described in US 4 289 513 and US 3340875.

Masking agents or deodourants such as perfumes may also be used as odour control agents herein. Preferably these agents are used in combination with an additional odour control agent such as zeolite as described in WO94/22500. Also so called anti perspirants such as aluminium salts for example aluminium chloridrate and aluminium sulphate and antimicrobics such as Triclosan and benzoic, propionic and sorbic acids for example may also be used as odour control agents. Such agents are described in "The Chemistry and Manufacture of Cosmetics" Vol. 3, 2 Ed. pg. 205-208, entitled "Antiperspirants and deodorants", by W. H. Mueller and R. P. Quatrale and "The Journal of Investigative Dermatology", Vol. 88, N. 3, March Suppl. 1987., entitled "Skin Microflora", by J. J. Leydon, K. D. McGinley et al.

Other suitable odour control agents are enzyme blocking agents as described in Cosm. and Toil. 95, 48, 1980, in " Non microbiological deodourising agents" by R. Osberghaus such as triethyl cytrate and odour absorbers for example zinc ricinoleate as described in Cosmesi Funzionale, pages 465-498, ED. Singerga, 1988, G. Proserpio.

The odour control system may be incorporated into the device by any of the methods disclosed in the art. For example, the odour control agents may be layered on the wall material or incorporated between the layers of the wall material. Alternatively, the odour control system may be contained within capsules or micro capsules which are activated by for example a change in temperature, pressure or by liquid contact on wall material. The odour control agents may be incorporated as a powder or a granulate within the device. For odour control systems comprising more that one component the agents may be granulated separately and then mixed together or granulated together. The odour control material can be particularly effectively incorporated in microporous and monolithic films during their production or alternatively can be sandwiched between the layers of the bag material.

Preferred odour control agents include carbon black, zeolites, silica, antimicrobial agents, perfuming ingredients, masking agents and chelants.

In one embodiment of the present invention the bag (11) may also contain absorbent material (15). The absorbent material (15) may comprise any absorbent material which is capable of absorbing and retaining liquids. The absorbent material (15) may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material (15) may be positioned in the bag (11) in any suitable manner. For example, the absorbent material (15) may be loosely arranged within the bag (11) or may be secured to the inner layer of the bag (11). Any known techniques for securing absorbent material (15) to nonwoven and film substrates may be used to secure the absorbent material to the inner layer of the bag (11). The absorbent material may also be arranged to have any desired shape or configuration (e.g., rectangular, oval, circular, etc.).

As shown in Figures 1 - 3 the bag (11) is provided with an aperture (21) whereby faecal matter is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction, most preferably the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange (12) is attached to the bag (11) by adhesive. Typically, the bag (11) will be attached to the flange (12), towards the outer periphery of flange so as not to cause any obstruction for the entering faecal matter.

The flange (12) may be provided in any size depending on the wearer group for which the device is intended. Similarly the flange (12) may be provided in any shape and preferably has a symmetrical shape preferably comprising a plurality of lobes (13).

In the embodiment shown in Figures 1-3, the outer surface of bag (11) is provided with patches of adhesive (40) for securing the bag (11) to the body of the wearer. Preferably, the patches of adhesive (40) are positioned on the outer surface of bag (11) such that they are secured to the abdomen of the wearer in use. Any number, size and shape of adhesive patches (40) may be used depending on the intended use of the device. The adhesive (40) may be any medically approved water resistant pressure sensitive adhesive such as hydrocolloid adhesives and hydrogel adhesives. Particularly effective adhesives in providing the desired adhesive properties to secure the flange to the skin of the wearer whilst allowing for relatively painless application and removal are hydrophilic hydrogels formed from crosslinking polymers with a plastisicer to form a 3-dimensional matrix.

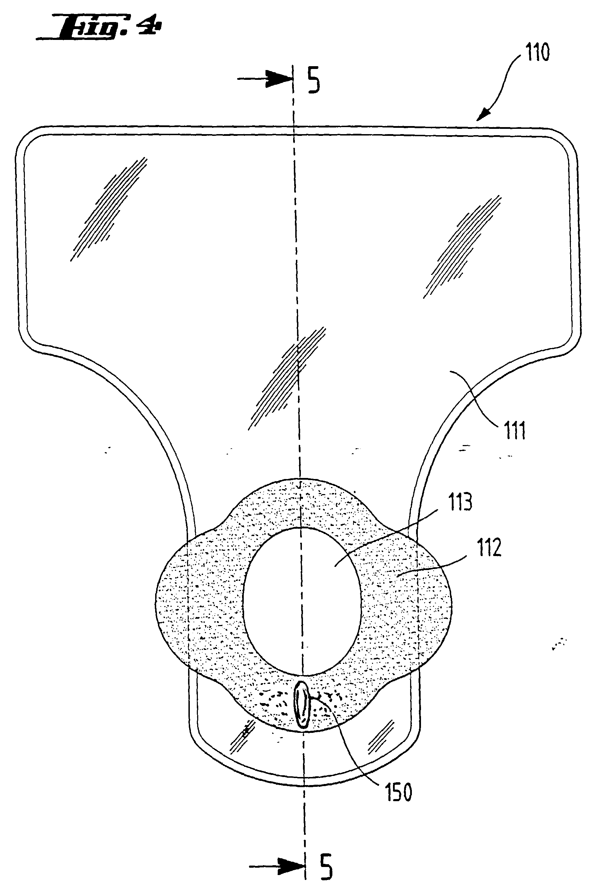

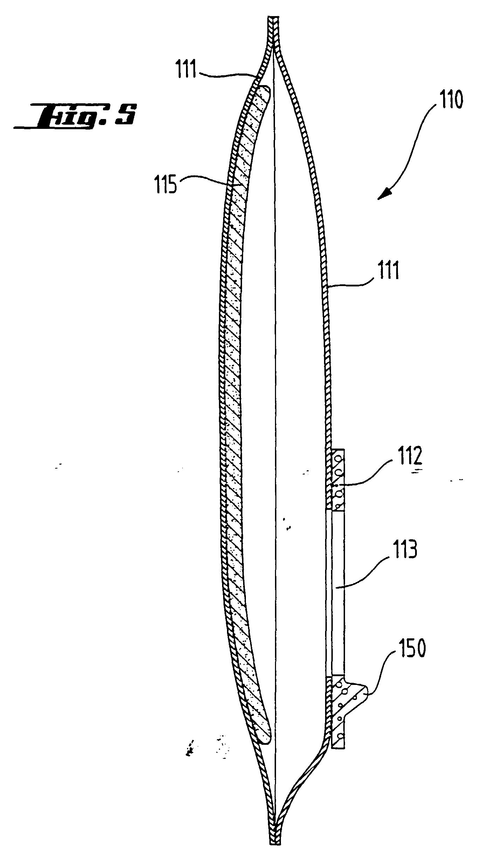

Referring now to Figures 4-5, there is shown another embodiment of a disposable urine management device (110). Disposable urine management device (110) comprises a bag (111) having an aperture (113), a flange (112) surrounding the aperture for adhesive attachment to the body of a wearer, and absorbent material (115) contained within the bag (111).

The flange (112) includes a raised, curved bulge (150) positioned beneath the aperture (113) and extending across the flange (112) for approximately the width of the aperture (113). The bulge (150) is shaped to span the perineum of the wearer.

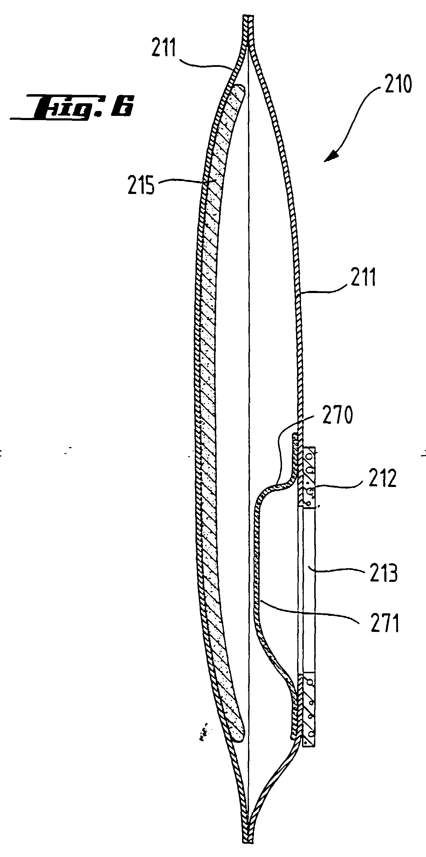

Referring now to Figure 6, there is shown another embodiment of a disposable urine management device (210). Disposable urine management device (210) comprises a bag (211) having an aperture (213), a flange (212) surrounding the aperture for adhesive attachment to the body of a wearer, and absorbent material (215) contained within the bag (211).

Disposable urine management device (210) also comprises an additional acquisition layer (270). Acquisition layer (270) is shown in Figure 6 to be secured to the inner surface of bag (211). However, the acquisition layer (210) may also be secured to the flange (212), or both the flange (212) and the inner surface of bag (211). Acquisition layer (270) is preferably positioned such that it separates the genitalia of the wearer from coming into direct contact with the absorbent material (215). Acquisition layer (270) is fluid pervious allowing urine to readily pass through so that it may be absorbed by absorbent material (215).

The acquisition layer (270) may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. If the acquisition, barrier layer includes fibers, the fibers may be spunbond, carded, wet-laid, meltblown, hydroentangled, or otherwise processed as is known in the art.

The acquisition layer (270) is designed to have a pore size such that the absorbent material (215) is not allowed to pass through and contact the wearer's skin. While designed not to have to large of a pore size which permits the passage of absorbent material (215), the acquisition layer (270) preferably has a pore size which is greater than the pore size of the absorbent material (215).

Preferably, the acquisition layer (270) is less hydrophilic than the absorbent material (215). The acquisition layer (270) may be treated with a surfactant to increase its initial wettability. When treated with surfactant, however, the acquisition layer (270) should still be less hydrophilic than the absorbent material (215). Suitable methods for treating the acquisition layer (270) with a surfactant include spraying the acquisition layer (270) with the surfactant and immersing the material into the surfactant. Alternatively, a surfactant may be incorporated into the acquisition layer (270).

As shown in Figure 1 the bag (11) is provided with an aperture (13) whereby urine is received from the body prior to storage within the bag cavity. The aperture (13) is surrounded by a flange (12) and may be provided in any shape or size, such as circular, oblong, heart shaped and may be symmetrical or asymmetrical, preferably the aperture has an oblong configuration either in the longitudinal or in the transversal direction, most preferably the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange (12) is attached to the bag (11) by adhesive. Typically, the bag (11) will be attached to the flange (12), towards the outer periphery of flange (12)so as not to cause any obstruction for the entering liquids.

The flange (12) may be provided in any size depending on the wearer group for which the device is intended. Similarly the flange (12) may be provided in any shape and preferably has a symmetrical shape preferably comprising a plurality of lobes (16).

The flange comprises a garment facing portion (21) and a wearer facing portion (22). In an preferred embodiment these are two large, substantially flat surfaces.

The flange (12) should be made of soft, flexible and malleable material to allow easy placement of the flange to the uro-genital area. In addition it is preferred that the flange (12) be made of a hydrophobic material such that if urine does come into contact with the perimeter (30) surrounding the aperture (13) it is repelled and does not wick to the outer edge (32) of the flange (12). Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 $g/m^2$, more preferably 50 $g/m^2$. Other thermoplastic foam materials, or other suitable plastics sheet materials having the described properties of such foams (i.e., softness, pliability, stretchability, and contractability) might also be used. Preferably, the material of garment facing surface (21) of the flange (12) may extend into the defined aperture area so as to form a skirt or flap of material which prevents unintentional adhesion of the surface edges of the flange (12) defining the aperture (13) to oneanother during use.

According to the present invention the urine management device (10) further comprises an attachment means to secure the device to the wearer. Such means include straps and more preferably comprises a body-compatible pressure sensitive adhesive (20) applied to the wearer facing portion (22) of the flange (12).

The adhesive (20) is preferably covered with a release means (not shown) in order to protect the adhesive (20) such as siliconized paper. The adhesive (20) can cover the entire wearer facing surface of the flange or more preferably have at least one, preferably two to six non-adhesive portions. These portions may be adhesive free or may contain inactivated or covered adhesives. As is evident from Figure 1, the adhesive is in one preferred embodiment not applied to the entire wearer facing surface area of the flange (12), so as to provide lobes (16) on either side of the flange (12) which are non-adhesive and can thereby serve to facilitate placement and removal of the device whilst avoiding contact with the adhesive. These lobes are however preferably also covered by the release means. Before application of the urine management device (10) to the skin of the wearer, the release means if present is removed. Alternatively a single lobe placed centrally about the longitudinal axis of the flange (12) is also particularly beneficial.

According to the present invention any medically approved water resistant pressure sensitive adhesive may be used to attach the device to the uro-genital area of the wearer, such as hydrocolloid adhesives and hydrogel adhesives. Particularly effective adhesives in providing the desired adhesive properties to secure the flange to the skin of the wearer at the sensitive uro-genital area, whilst allowing for relatively painless application and removal, are formed from crosslinking polymers with a plastisicer to form a 3-dimensional matrix.

Suitable adhesives for use herein are hydrogel adhesives available from 3M and Promeon.

The adhesive (20) can be applied to the wearer facing surface (22) of the flange (12) by any means known in the art such as slot coating, spiral, or bead application or printing. Typically the adhesive (20) is applied at a basis weight of from $20g/m^2$ to $2500g/m^2$, more preferably from $500g/m^2$ to $2000g/m^2$ most preferably from $700g/m^2$ to $1500g/m^2$ depending on the end use envisioned. For example for urine management devices (10) to be used for babies the amount of adhesive (20) may be less than for urine management devices designed for active adult incontinence sufferers.

According to another aspect of the present invention, the urine management device (10) has been found particularly useful and beneficial when utilized in conjunction with a garment or a disposable diaper and/or a faecal management device. Typically the urine management device will be positioned to the uro-genital area of the wearer positioned and secured to the wearer by the adhesive flanges and patches of adhesives. Subsequently, the diaper is positioned over the urine management device (10) and fastened in a conventional manner around the body of the wearer. It has been found that in addition to providing excellent separation between urine and faecal matter, the combined urine management device (10) and diaper system reduce skin irritation, which may at time occur, especially as the wearer group includes the very old, young and unhealthy wearers.

## Test methods

### Air and Vapour Permeability Test

The Vapour permeability test is utilised to quantify the vapour transmission properties of breathable bag materials of urine management devices.

### Basic Principle of the Methods

The basic principle of the test is to quantify the extent of water vapour transmission through the wall of the bag of the urine management device. The test method that is applied is based on a standardized textile industry applied test method and commonly referred to as the "cup test method". The test is performed in a stable temperature/humidity laboratory maintained at a temperature of 23° C at 50% RH for a period of 24 hours.

### Apparatus

    1) Sample cup of dimensions (open area = 0.003 $m^2$)
    2) Syringe to introduce the distilled water into the completed sample cup.
    3) Wax to seal the cup once sample has been arranged.
    4) A circular punch to facilitate preparation circular samples of diameter = 30 mm.
    5) Laboratory of stable climatic conditions (230 C $\pm$ 0.5°C / 50% RH $\pm$ 1 % RH)
    6) Laboratory balance accurate to 4 decimal places.

## Sample Preparation / Measurements

The test is to be performed on the wall material of the bag. A representative device is selected and a sample is cut to size using the punch. The sample cut is sufficiently large to adequately overlap the sample holder and to ensure material that may have been damaged or undesirably stretched due to the cutting operation lies outside of the measurement centre when the measurement is performed. The sample is so arranged onto of the sample cup so as to fully overlap the cup. The sample is oriented so as to ensure that the surface exposed to the laboratory environment is the same that would be found while wearing the article.

The closure ring of the sample cup is then placed onto the sample and pushed down. This ensures that the excess material is held firmly in place and does not interfere with the measurement. A wax is then applied to the entire surface of the closure ring to ensure the whole upper part of the apparatus is closed to the environment. Distilled water (5 ± 0.25 ml) is introduced with the syringe into the sealed sample cup via the minute perforation. Finally this perforation is sealed with silicone grease.

The entire cup (containing sample and water) is weighed and the weight recorded to 4 decimal places. The cup is then placed in a ventilation stream generated by a fan. The air flowing over the top of the sample cup is 3 ±0.3 m/sec and confirmed via a wind velocity meter ("Anemo", supplied by Deuta SpA., Italy). The sample cup remains in the ventilated test field for a period of 24 hrs and is then re-weighted. During this period if the test sample is sufficiently breathable the liquid in the sample holder is able to diffuse out of the sample holder and into the laboratory environment. This results in a reduction in the weight of water in the sample holder that can be quantified on re-weighing the complete sample cup following the 24 hr period.

The vapour permeability value is determined as the weight loss divided by the open area of the sample holder and quoted per day.

$$i.e.\ Vapour\ Permeability = Weight\ Loss\ (g)/(0.003\ m^2/24\ hrs)$$

## Air Permeability test

The air permeability test is utilised to assess the ability of wall material of the bag of urine management devices to circulation/exchange air.

## Basic Principle of the Methods

The basic principle of the test is to evaluate the resistance of the wall material to the passage of air. In this test, the volume (or amount) of air that flows through an article of given dimensions under standard conditions (of 23 °C /50% RH) is measured. The instrument utilised for the test is: Air Permeabilimeter FX 3300 manufactured by TexTest AG Switzerland.

Samples should be allowed to equilibrate in the test environment for at least 4 hrs prior to commencement of the measurement. The article (having dimensions exceeding 5 $cm^2$ the dimensions of the measurement head) is placed on the device as instructed by the manufacturer. An aspiration pump set to generate a pressure of 1210 kPa that sucks air through the sample layer or structure. The device measures the volume of air flow and the pressure drop across the orifices that contains the sample and measurement head. Finally the device generates a value of air permeability in the units of "$l/M^2s^1$"

## Wet-Through Test

The wet-through test is utilised to evaluate the resistance of a the bag wall material or construction to the transmission of bodily discharges. It can be used as a direct measure of how liquid-impervious the porous wall material is to the full range of bodily discharges by simply changing the composition of the test solution as will be detailed following the method description.

## Basic Principle of the Methods

The basic principle of the test is to simulate the loading of a faecal management device in-use with bodily discharges. To achieve this a sample device is prepared, and held on in position in a test stand clapped about the flange such that the outer surface of the wall material of the bag of the garment facing surface is in contact with the test stand (bottom side). Suspended above the sample to be analysed is a liquid delivery system that is capable of delivering any desired quantity of the desired test liquid (either as a burst or as a series of steps as is desired).

Located between the garment facing surface of the bag wall material of the test sample and the transparent test stand is a sheet of absorbent filter paper. This absorbent filter paper is in intimate contact with the wall material of the test sample to simulate, for example a when the faecal management device is in close contact with the clothing or a diaper. Directly below the transparent test stand is a mirror so positioned to allow any change in the absorbent filter paper (wetting with coloured solutions simulating bodily discharges) to be continuously observed. For example if the porous bag wall material is unable to adequately resist liquid transmission then the filter paper will become wet with the coloured solution and this can be observed in the mirror. The magnitude of the transmitted solution either as a weight or more preferable the size of the stain on the absorbent filter paper (simulating the undergarment or diaper) in addition to the time dependence of the transmission can be readily recorded.

The test solution is introduced to the test sample via a calibrated delivery system such as via a simple burette according to the desired test approach as detailed below. Once the bag has been loaded with the test solution, a period of one (1) minute is allowed for the system to reach equilibrium.

Following the one minute wait the test sample is placed under a pressure of 70 gsm (grams per square meter) which is believed to reflect more stressful pressures that are nevertheless regularly obtained in-use. The test sample remains under the 70 gsm pressure for a period of up to 30 min. and measurements, for example the area of the coloured stain on the absorbent paper, is measured at 5 minute intervals.

It is also important to understand the mechanism of wet-through failure and to ensure the exact test design is able to correctly assess this. For example a breathable bag wall material with relatively large apertures (> 200 $\mu$m) is more likely to fail due to a process of extrusion (such as when sitting the pressure exerted may force the liquid through the relatively large apertures) which will happen relatively quickly on placing the test sample under pressure. Alternatively as the apertures are made ever smaller (<200 $\mu$m) a process of simple diffusion or capillary driven diffusion is more likely to occur. Such process are slow compared to extrusion processes.

This test design measures the imperviousness of the porous wall material under a high loading (sudden stressful gush of test solution) simulation.

The high gush simulation test is performed as detailed in the above general description under the following conditions for a faecal management device:

Test Solution: Synthetic Urine + 1 %Surfactant
Gush Volume (ml): 10 ml
Gush Rate (ml/min.): 10 (i.e. 10 ml in 60 seconds)
Pressure applied: 70 gsm (after 1 min. wait)

Results reported as area of stain/wet-through in units of square cm ($cm^2$) at time elapses of 5, 10, 20 and 30 mins.

Test solution type and volumes utilised in the test methods.

In order to reliably assess the potential bag wall materials the test solution conditions should be matched to the product end use. These discharges can be quite varied for different wearers and may contain various levels of fatty acids and detergent type contaminants from daily hygienic practices (washing, laundering etc.). These components are extremely mobile and may have very low surface tensions. Due to the bodily contaminants (fatty acids, surfactants and detergent residues) found it has been determined that the addition of surfactant to a synthetic urine solution correlates well to conditions found in use. The volumes again are chosen to reflect typical conditions that this application is likely to expose the products to. For more stressful applications the methods can be readily modified to simulate higher test solution loading volumes and rates of delivery.

Preparation of Test Solution Synthetic Urine + 1 % Surfactant (UreaB/1%).

The test solution Synthetic Urine is first prepared in a 10 kg master batch and smaller quantities are removed as required and surfactant is added. Each 10 Kg UreaB batch is composed of the following components:

| Component: | Formula | Quantity/10Kg batch |
|---|---|---|
| Urea | | 200 g |
| Sodium Chloride | NaCl | 90 g |
| Magnesium Sulphate | $MgSO_4.7H_2O$ | 11 g |
| Calcium Chloride | $CaCl_2$ | 6 g |
| Distilled Water | $H_2O$ | 9693 g |

All reagents are "Reagent Grade" and available from standard Chemical suppliers. Additionally surfactant is supplied by Pegesis, U.S.A, Peosperse 200ML. For individual measurements typically a 100 ml. test solution UreaB/1% Surfactant is prepared by mixing 90 ml. Urea B solution with 10 ml. Surfactant. The UreaB/1% solution must be constantly mixed to ensure the components do not separate prior to usage.

**Claims**

1.  A urine management device (10) comprising a bag (11), said bag (11) having an aperture (13) and a flange (12) surrounding said aperture for adhesive attachment to uro-genital area of the wearer, said flange (12) being attached to said bag (11), and said bag (11) comprising a wall material, characterised in that said wall material is moisture vapour permeable and has a moisture vapour transport rate of at least 250 g/(m$^2$.24hrs.).

2.  A urine management device (10) according to claim 1, wherein said wall material has a moisture vapour transport rate of at least 300 g/(m$^2$.24hrs.).

3.  A urine management device (10) according to claim 2, wherein said wall material has an air permeability of at least 50 l/(m$^2$.s)

4.  A urine management device (10) according to any of the preceding claims, wherein said wall material comprises at least one layer selected from monolithic films and microporous films.

5.  A urine management device (10) according to any one of the preceding claims, wherein said wall material comprises as inner layer (18) and outer layer (17), wherein said outer layer (17) is a nonwoven layer and said inner layer (18) is a monolithic film, and wherein said inner layer (18) and outer layer (17) are laminated.

6.  A urine management device according to any one of the preceding claims, wherein said wall material further comprises an odour control agent.

7.  A urine management device (10) according to claim 6, wherein said odour control agent is selected from silica, zeolites, chelating agents, active carbon, and mixtures thereof.

8.  A urine management device (10) according to any one of the preceding claims, wherein said bag (11) contains absorbent material (15) therein.

9.  The use of a urine management device (10) according to any of the preceding claims in combination with a disposable diaper.

## Fig. 1

Fig. 2

Fig. 3

_Fig. 4_

Fig. 5

Fig. 6

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | GB 2 188 855 A (CRAIG MED PROD LTD) 14 October 1987 (1987-10-14) * the whole document * | 1-8 | A61F5/44 |
| A | EP 0 273 611 A (GRACE W R & CO) 6 July 1988 (1988-07-06) * column 4, line 36 - line 49 * | 1 | |
| A | WO 97 01316 A (BAUTISTA MORENO MARIA LUCIA ;VALLADARES PEREDA VICTORIANO (ES)) 16 January 1997 (1997-01-16) | | |

**TECHNICAL FIELDS SEARCHED (Int.Cl.6)**

A61F

The present search report has been drawn up for all claims

| Place of search | Date of completion of the search | Examiner |
|---|---|---|
| THE HAGUE | 4 October 1999 | Sánchez y Sánchez, J |

CATEGORY OF CITED DOCUMENTS

X : particularly relevant if taken alone
Y : particularly relevant if combined with another document of the same category
A : technological background
O : non–written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 0 966 933 A1**

**ANNEX TO THE EUROPEAN SEARCH REPORT
ON EUROPEAN PATENT APPLICATION NO.**   EP 98 12 4492

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-1999

| Patent document cited in search report | | Publication date | Patent family member(s) | | Publication date |
|---|---|---|---|---|---|
| GB 2188855 | A | 14-10-1987 | NONE | | |
| EP 0273611 | A | 06-07-1988 | AR | 240279 A | 30-03-1990 |
| | | | AT | 60866 T | 15-02-1991 |
| | | | AU | 606109 B | 31-01-1991 |
| | | | AU | 8300487 A | 30-06-1988 |
| | | | CA | 1320324 A | 20-07-1993 |
| | | | DK | 684887 A,C | 25-06-1988 |
| | | | JP | 2622391 B | 18-06-1997 |
| | | | JP | 63168347 A | 12-07-1988 |
| | | | KR | 9512179 B | 14-10-1995 |
| | | | MX | 169682 B | 19-07-1993 |
| | | | NZ | 223023 A | 28-11-1989 |
| | | | US | 4906495 A | 06-03-1990 |
| | | | US | 5110390 A | 05-05-1992 |
| | | | ZA | 8709620 A | 23-06-1988 |
| WO 9701316 | A | 16-01-1997 | ES | 1032931 U | 16-07-1996 |
| | | | AU | 6127396 A | 30-01-1997 |

EPO FORM P0459

For more details about this annex : see Official Journal of the European Patent Office, No. 12/82

20